# EUROPÄISCHE PATENTSCHRIFT

(11) **EP 3 223 882 B1**
(45) Veröffentlichungstag und Bekanntmachung des Hinweises auf die Patenterteilung: **29.08.2018**
(21) Anmeldenummer: 15770806.6
(22) Anmeldetag: 25.09.2015
(51) Int. Cl.: A61M 1/14, A61M 1/16, A61M 1/26, A61M 1/34, A61M 1/36

(54) **DIALYSEMASCHINE**
DIALYSIS MACHINE
MACHINE DE DIALYSE

(43) Veröffentlichungstag der Anmeldung: 04.10.2017
(73) Patentinhaber: Fresenius Medical Care Deutschland GmbH, 61352 Bad Homburg (DE)
(72) Erfinder: GAGEL, Alfred, 96123 Litzendorf (DE); KOPPERSCHMIDT, Pascal, 97456 Dittelbrunn (DE); NÜRNBERGER, Thomas, 97705 Burkardroth (DE)
(74) Vertreter: Herrmann, Uwe
(86) Internationale Anmeldenummer: PCT/EP2015/001909
(87) Internationale Veröffentlichungsnummer: WO 2016/045798

(56) Entgegenhaltungen:
- WO-A1-03/028860
- WO-A1-2009/144522
- US-A1- 2014 083 943

## Beschreibung

Die Erfindung betrifft eine Dialysemaschine mit einem extrakorporalen Blutkreislauf, einer Blutpumpe, einem Dialysator, einem Drucksensor zur Messung des Flüssigkeitsdrucks im extrakorporalen Blutkreislauf, einer Leitung für die Zufuhr von Substitutionsflüssigkeit in den extrakorporalen Blutkreislauf und einer Steuereinheit.

Im Stand der Technik sind Dialyseverfahren bekannt, in denen dem Patientenblut aufgrund einer Flüssigkeitskonvektion durch die Dialysatormembran ein bestimmtes Plasmavolumen entzogen wird. Zu diesen Verfahren zählen die Hämodiafiltration und die Hämofiltration. Um in diesen Verfahren zu verhindern, dass eingedicktes Blut an den Patienten rückgeführt wird, wird fortlaufend Substitutionsflüssigkeit (Substituat) in den extrakorporalen Blutkreislauf zugeführt.

Unterbleibt oder verringert sich aus bestimmten Gründen die Zufuhr von Substituat zumindest temporär, so fließt eingedicktes Blut durch die venöse Leitung des extrakorporalen Blutkreislaufs und den venösen Anschluss. Häufige Gründe für ein zumindest temporäres Unterbleiben bzw. eine Verringerung der Zufuhr von Substituat umfassen z.B. eine Änderung der UF-Rate oder eine Umstellung von Hämodiafiltration auf Hämodialyse bei laufender Behandlung. Weitere Gründe umfassen z.B. die Durchführung eines Druckhaltetests, eine Sicherheitsabschaltung der Substituatzufuhr oder einen Tausch der Konzentratbehälter.

Durch die höhere Viskosität des eingedickten Blutes steigt temporär der Flüssigkeitsdruck (d.h. der Blutdruck) im extrakorporalen Blutkreislauf. Dies kann beispielsweise zu einem Druckalarm führen und ist unerwünscht.

Vor diesem Hintergrund ist aus der WO 2009/144522 A1 bekannt, neben dem Normalbetrieb der Blutpumpe, in dem die Blutpumpe z.B. mit einer bestimmten Sollförderrate betrieben wird, einen speziellen Betriebsmodus der Blutpumpe vorzusehen, in dem die Förderrate in Abhängigkeit des venösen Drucks geregelt wird. Der spezielle Betriebsmodus startet nach einem Triggerereignis und endet nach Ablauf einer bestimmten Zeitdauer. Das Triggerereignis steht im Zusammenhang mit einer Reduktion der Substituatzufuhr. Beispielsweise liegt ein Triggerereignis vor, wenn der venöse Druck, die Förderrate der Substituatpumpe oder die Änderung dieser Größen einen bestimmten Schwellenwert über- oder unterschreitet. Auf diese Weise kann in dem speziellen Betriebsmodus erreicht werden, dass sich die Förderrate der Blutpumpe temporär verringert, wenn ein Schwellenwert für den venösen Druck überschritten wird oder droht, überschritten zu werden.

Ein weiteres Verfahren zum Betreiben einer Dialysemaschine ist aus der WO 03/028860 A1 bekannt. Die darin beschriebene Offenbarung geht aber nicht über die Merkmale aus der Präambel des Anspruchs 1 hinaus.

Die US 2014/083943 A1 zeigt ebenfalls ein Verfahren zum Betreiben einer Dialysemaschine, das zumindest die Merkmale aus dem kennzeichnenden Teil des Anspruchs nicht thematisiert.

Bei einer derartigen Regelung können jedoch dann Probleme auftreten, wenn ein weiteres Triggerereignis noch während des speziellen Betriebsmodus auftritt oder wenn zwischen dem Ende des speziellen Betriebsmodus und den nächsten Triggerereignis nur wenig Zeit vergeht. Denn der Sollwert des venösen Drucks, an dem sich der tatsächliche venöse Druck während des speziellen Betriebsmodus orientiert, leitet sich aus dem gemessenen venösen Druck vor dem Triggerereignis ab. Dieser kann im speziellen Betriebsmodus durch eine gewisse Trägheit des Systems größeren Schwankungen unterworfen sein als im Normalbetrieb und stabilisiert sich nach Ende des speziellen Betriebsmodus erst nach Ablauf einer gewissen Zeit. So kann ein ungeeigneter Sollwert angenommen werden, sollte es in dieser Phase zur Auslösung einer weiteren Episode des speziellen Betriebsmodus kommen.

Dies kann beispielsweise zu einer wiederholten Selbsttriggerung des speziellen Betriebsmodus führen. Denn wenn im speziellen Betriebsmodus ein falscher Sollwert angenommen wird, weicht die Förderrate der Blutpumpe stark von der Sollförderrate für den Normalbetrieb ab und ändert sich nach Ende des speziellen Modus sprunghaft. Diese Änderung der Förderrate kann ein weiteres Triggerereignis mit sich bringen, beispielsweise einen kurzen Peak im venösen Druck oder - sofern diese an die Förderrate der Blutpumpe gekoppelt ist - in der Förderrate der Substituatpumpe, und die erneute Annahme eines falschen Sollwerts für den venösen Druck im speziellen Modus ist wahrscheinlich.

Eine Auswirkung kann ferner sein, dass es häufig zur Auslösung eines Druckalarms kommt, da bei einer Regelung um einen falschen Sollwert häufiger ein im Normalmodus ermittelter Grenzwert zur Auslösung des Alarms unter- oder überschritten wird.

Aufgabe der vorliegenden Erfindung ist es, das Risiko zu reduzieren, dass im speziellen Modus eine ungeeignete Vorgabe für eine Steuerung bzw. ein ungeeigneter Sollwert für eine Regelung der Blutpumpe angenommen wird.

Vor diesem Hintergrund betrifft die Erfindung eine Dialysemaschine, die einen extrakorporalen Blutkreislauf, eine Blutpumpe, einen Dialysator, einen venösen Drucksensor zur Messung des venösen Drucks im extrakorporalen Blutkreislauf und eine Leitung für die Zufuhr von Substituat in den extrakorporalen Blutkreislauf aufweist. Die Dialysemaschine weist ferner eine Steuereinheit auf, die ausgebildet ist, die Blutpumpe in einem ersten Betriebsmodus und einem speziellen Betriebsmodus zu betreiben und nach Erkennung eines Triggerereignisses den speziellen Betriebsmodus zu starten. In dem speziellen Betriebsmodus wird eine Förderrate der Blutpumpe anhand einer Vorgabe gesteuert oder auf einen Sollwert geregelt, wobei die Vorgabe bzw. der Sollwert einem vor Beginn des aktuell gestarteten bzw. durch das aktuelle Triggerereignis zu startenden speziellen Betriebsmodus ermittelten Wert entspricht bzw. daraus abgeleitet wird.

Erfindungsgemäß ist vorgesehen, dass die Steuereinheit ferner so ausgebildet ist, dass vor Start des speziellen Betriebsmodus das Vorliegen wenigstens eines Hinderungsgrundes abgefragt wird. Bei Vorliegen des Hinderungsgrundes wird der Start des speziellen Betriebsmodus blockiert oder verschoben. Alternativ oder zusätzlich weicht bei Vorliegen des Hinderungsgrundes die Wahl der Vorgabe bzw. des Sollwertes von der korrespondierenden Wahl im hypothetischen Falle eines fehlenden Vorliegens des Hinderungsgrundes ab.

Der vor Beginn des aktuell gestarteten speziellen Betriebsmodus ermittelte (Mess-) Wert, der der Vorgabe bzw. dem Sollwert entspricht bzw. aus dem die Vorgabe bzw. der Sollwert abgeleitet wird, kann sich in einer Ausführungsform auf eine Bluteigenschaft beziehen. Beispiele umfassen eine Blutviskosität oder ein Hämatokritanteil. In einer weiteren Ausführungsform kann es sich bei dem Messwert, auf dessen Grundlage die Vorgabe bzw. der Sollwert bestimmt wird, um einen Gerätewert handeln. Beispiele umfassen eine Motorstromaufnahme oder Motorleistungsaufnahme der Blutpumpe. Ein bevorzugtes Beispiel umfasst den venösen Druck. Der Sollwert bzw. die Vorgabe, die aus diesem Wert abgeleitet werden, können ebenfalls diese Parameter umfassen. Auch das Ableiten einer Vorgabe bzw. eines Sollwertes in Form einer dieser Parameter, beispielsweise einer Motorleistungsaufnahme, aus einem Messwert in Form eines anderen dieser Parameter, z.B. des venösen Blutdrucks, ist denkbar und von der Erfindung umfasst.

Beispielsweise kann vorgesehen, dass die Förderrate der Blutpumpe im speziellen Betriebsmodus erniedrigt wird, wenn der gemessene venöse Druck den Sollwert übersteigt, und dass die Förderrate der Blutpumpe im speziellen Betriebsmodus erhöht wird, wenn der gemessene venöse Druck unter dem Sollwert liegt. Alternativ kann vorgesehen sein, dass die Steuereinheit zur Steuerung der Blutpumpe im speziellen Betriebsmodus eine Vorgabe verwendet, die einem Wert entspricht oder aus einem Wert abgeleitet wurde, der unmittelbar vor Einsetzen des speziellen Modus gemessen bzw. ermittelt wurde (z.B. venöser Druck oder Motorstromleistungsaufnahme).

In einer Ausführungsform besteht ein Hinderungsgrund darin, dass die Blutpumpe aktuell bereits im speziellen Modus betrieben wird. Sofern in diesem Fall die Wahl der Vorgabe bzw. des Sollwertes bei Vorliegen des Hinderungsgrundes vom Normalfall abweicht, kann die Vorgabe bzw. der Sollwert bei Vorliegen des Hinderungsgrundes aus dem bestehenden speziellen Betriebsmodus übernommen werden.

In einer Ausführungsform besteht wenigstens einer der Hinderungsgründe darin, dass eine bestimmte Karenzzeit nach Start oder Ablauf einer zeitlich vorgelagerten Phase, in der die Blutpumpe im speziellen Betriebsmodus betrieben wurde, noch nicht erreicht wurde. Sofern in diesem Fall die Wahl der Vorgabe bzw. des Sollwertes bei Vorliegen des von der Wahl ohne Vorliegen des Hinderungsgrundes abweicht, kann die Vorgabe bzw. der Sollwert aus dem speziellen Betriebsmodus während der zeitlich vorgelagerten Phase übernommen werden. Die Dauer der Karenzzeit kann der prognostizierten Flusszeit des Blutes durch die venöse Leitung des extrakorporalen Blutkreislaufs entsprechen oder sich daran, z.B. mit einem prozentuellen Abschlag oder Zuschlag (10%, 20%, 30%, 50% etc.), orientieren. Ferner ist denkbar, dass die Karenzzeit starr durch eine Benutzereingabe oder maschinenseitige Vorgabe festgelegt wird oder an einen bestimmten Volumendurchsatz der Blutpumpe angelehnt wird.

Alternativ zum Ende der zeitlich vorgelagerten Phase kann als Startpunkt der Karenzzeit auch der Beginn der zeitlich vorgelagerten Phase verwendet werden. Ferner ist denkbar, dass als Startpunkt einer Karenzzeit ein Ereignis der Behandlung verwendet wird, das von einer zeitlich vorgelagerten Phase, in der die Blutpumpe im speziellen Betriebsmodus betrieben wurde, unabhängig ist. Darunter fällt beispielsweise ein Zeitpunkt, in dem ein Messwert (z.B. beispielsweise die erste zeitliche Ableitung des venösen Drucks) einen Schwellenwert überschreitet oder in dem ein gewisser Abstand eines Messwerts (z.B. des venösen Drucks) von einem Erwartungswert vorliegt.

In einer Ausführungsform besteht ein Hinderungsgrund darin, dass die erste zeitliche Ableitung des venösen Drucks bei Vorliegen des Triggerereignisses einen Schwellenwert überschreitet. Dies ist gleichbedeutend mit einem instabilen Zustand, in dem sich keine geeignete Vorgabe bzw. kein geeigneter Sollwert ermitteln lässt. Auch in diesem Fall wird entweder die Auslösung des speziellen Betriebsmodus blockiert oder die Vorgabe bzw. der Sollwert wird aus dem zeitlich vorgelagerten speziellen Betriebsmodus wird für den neuen speziellen Betriebsmodus angenommen.

Eine Verschiebung der Auslösung des speziellen Betriebsmodus ist ein Unterfall der Blockierung, denn eine Blockierung der Auslösung kann zu einer Verschiebung der Auslösung führen, wenn das Triggerereignis bestehen bleibt (und periodisch oder kontinuierlich weiterhin abgefragt wird) und der Hinderungsgrund wegfällt.

Mehrere Hinderungsgründe können kumulativ abgefragt werden. Ferner ist denkbar, dass lediglich ein oder zwei Hinderungsgründe abgefragt werden.

In einer Ausführungsform wird die Vorgabe bzw. der Sollwert aus dem Messwert, beispielsweise dem venösen Druck vor Starten des speziellen Modus abgeleitet. Beispielsweise kann ein Messwert des venösen Drucks innerhalb der letzten 60, 30 oder 10 Sekunden vor Auftreten des Triggerereignisses bzw. Start des speziellen Betriebsmodus angenommen werden, gegebenenfalls mehr als 1 Sekunde vor oder auch weniger als 1 Sekunde vor Auftreten des Triggerereignisses bzw. Start des speziellen Betriebsmodus. Auch die Annahme eines Mittelwertes aus mehreren in diesem Zeitraum ermittelten Messwerten ist denkbar.

In einer Ausführungsform wird die Blutpumpe vor und/oder nach dem speziellen Betriebsmodus mit einem vorgegebenen Flussratenverlauf, insbesondere bei konstanter Flussrate oder konstanter Drehzahl betrieben.

Das Triggerereignis kann beispielsweise in der Über- oder Unterschreitung eines Erwartungswertes für den Messwert, beispielsweise den venösen Druck liegen. Dies kann beispielsweise im Zusammenhang mit einer Erniedrigung der Ultrafiltationsrate oder mit einer Umstellung von Hämodiafiltration auf Hämodialyse stehen, wenn die Zugabe von Substituat gedrosselt bzw. eingestellt wird, sich aber in einem Leitungsabschnitt stromaufwärts der Mündungsstelle für die Substituatleitung und stromabwärts des Dialysators noch eingedicktes Blut befindet. Ferner kann dies im Zusammenhang mit einer Unterbrechung der Substituatzufuhr stehen, beispielsweise im Rahmen einer Durchführung eines Druckhaltetests des Substituatkreislaufs, einer Sicherheitsabschaltung der Substituatzufuhr (z.B. Detektion einer Leitfähigkeitsschwankung oder von Luftblasen im Substituat) oder eines Tauschs der Konzentratbehälter. Der venöse Druck kann erniedrigt sein, wenn sich stärker verdünntes Blut in wenigstens einem Abschnitt der venösen Leitung befindet. Dies kann beispielsweise im Zusammenhang mit einer Erhöhung der Ultrafiltationsrate bzw. des Transmembrandrucks oder mit einer Umstellung von Hämodialyse auf Hämodiafiltration stehen, wenn die Zugabe von Substituat erhöht bzw. gestartet wird, sich aber in einem Leitungsabschnitt stromaufwärts der Mündungsstelle für die Substituatleitung und stromabwärts des Dialysators noch weniger stark eingedicktes Blut befindet. Ferner kann dies im Zusammenhang mit einer Bolusgabe von Substituat in den extrakorporalen Blutkreislauf stehen, um beispielsweise bestimmte Messverfahren durchführen zu können.

Das Triggerereignis kann ferner in einem Stopp, einer Verringerung, einem Start oder einer Erhöhung der Substituatgabe liegen. Dies kann im selben Zusammenhang stehen, wie dies oben mit dem Triggerereignis der Über- oder Unterschreitung eines Schwellenwertes für den venösen Druck diskutiert wurde. Ferner ist die Förderrate einer in der Substituatleitung befindlichen und für die Förderung des Substituats in den extrakorporalen Blutkreislauf verantwortlichen Substituatpumpe oftmals an die Förderrate der Blutpumpe gekoppelt, sodass auch behandlungsbedingte Änderungen der Förderrate der Blutpumpe ein derartiges Ereignis auslösen können.

Der spezielle Betriebsmodus kann solange andauern, bis er manuell vom Benutzer beendet wird. Alternativ oder zusätzlich kann vorgesehen sein, dass die Dauer des speziellen Betriebsmodus an die prognostizierte Flusszeit des Blutes durch die venöse Leitung des extrakorporalen Blutkreislaufs unter Normalbetrieb der Blutpumpe angenähert wird oder sich daran orientiert. Beispielsweise kann diese Dauer angenommen werden oder es kann ein Sicherheitszuschlag von mindestens 10%, 20 % oder 50%, aber ggf. von weniger als 100% hinzugefügt werden. Eine Prognose der Flusszeit des Blutes durch die venöse Leitung des extrakorporalen Blutkreislaufs unter Normalbetrieb der Blutpumpe kann beispielsweise auf der Grundlage der Förderrate der Blutpumpe vor Eintritt des speziellen Betriebsmodus und der bekannten Bauart des extrakorporalen Blutkreislaufs (Länge, Durchmesser, Gesamtvolumen der einzelnen Abschnitte, etc.) erfolgen. Typische prognostizierte Zeitdauern umfassen beispielsweise Zeitdauern von zwischen 1 und 10 Minuten oder von zwischen 10 und 60 Sekunden oder auch von zwischen 1 und 10 Sekunden.

Denkbar ist es weiterhin, dass die Steuereinheit so ausgebildet ist, dass die Karenzzeit mit der Dauer des speziellen Betriebsmodus korreliert.

In einer Ausführungsform mündet die Leitung für die Zufuhr von Substituat in der venösen Leitung des extrakorporalen Blutkreislaufs. Es handelt sich also um eine Leitung für die Postdilution. Die Leitung kann stromaufwärts oder stromabwärts des Drucksensors in den extrakorporalen Blutkreislauf münden. Alternativ kann auch vorgesehen sein, dass die Leitung für die Zufuhr von Substituat in der arteriellen Leitung des extrakorporalen Blutkreislaufs mündet. Die durch Schwankungen in der Prädilution hervorgerufenen Änderungen des venösen Drucks sind aber generell geringer als die durch Schwankungen in der Postdilution hervorgerufenen Änderungen des venösen Drucks.

Die Leitung zur Zuführung des Substituats kann eine Substituatpumpe umfassen, die für die Förderung des Substituats in den extrakorporalen Blutkreislauf verantwortlich ist.

In einer Ausführungsform wird während des Verlaufs des speziellen Betriebsmodus eine Veränderung in der Zufuhrrate des Substituats blockiert oder nur innerhalb eines vorgegebenen Bereichs zugelassen. Diese Maßnahme kann das Auftreten von Triggerereignissen während des speziellen Modus verhindern, beispielsweise ein Unterschreiten der Druckgrenzen. Beispielsweise kann auf diesem Weg eine Bolusgabe während des speziellen Modus blockiert werden. Ferner kann diese Maßnahme das Auftreten von Triggerereignissen zu Ende des speziellen Modus verhindern. Denn eine Erhöhung der Substitutionsrate während des speziellen Betriebsmodus führt durch die Blutverdünnung zu einem geringeren Druck und einer Erhöhung der Förderrate der Blutpumpe. Diese Förderrate würde zu Ende des speziellen Betriebsmodus dann ruckartig geändert.

In einer Ausführungsform wird innerhalb einer gewissen Wartezeit nach Ende des speziellen Betriebsmodus eine Veränderung der Zufuhr des Substituats blockiert oder nur innerhalb eines vorgegebenen Bereichs zugelassen. Diese Maßnahme kann das Auftreten von Triggerereignissen unmittelbar nach Ende des speziellen Modus verhindern. Auch das Auftreten von unerwünschten Druckalarmen kann verhindert werden, denn große Veränderungen des Verhältnisses der Substitutionsrate zu Blutfluss werden verhindert. Die Dauer der Wartezeit kann der prognostizierten Flusszeit des Blutes durch die venöse Leitung des extrakorporalen Blutkreislaufs entsprechen oder sich daran, z.B. mit einem prozentuellen Abschlag oder Zuschlag (10%, 20%, 30%, 50% etc.), orientieren. Ferner ist denkbar, dass die Wartezeit starr durch eine Benutzereingabe oder maschinenseitige Vorgabe festgelegt wird oder an einen bestimmten Volumendurchsatz der Blutpumpe angelehnt wird.

Die erfindungsgemäße Ausbildung der Steuereinheit beruht darauf, dass die Steuereinheit mit den relevanten Bauteilen der Dialysemaschine verbunden ist und dass in der Steuereinheit ein Algorithmus hinterlegt ist, der die erfindungsgemäße Ansteuerung der Blutpumpe ermöglicht.

Weitere Einzelheiten und Vorteile der Erfindung ergeben sich aus den nachfolgend diskutierten Figuren und Ausführungsbeispielen. In den Figuren zeigen:
- Figur 1:: eine schematische Darstellung einer erfindungsgemäßen Dialysemaschine;
- Figur 2:: eine schematische Darstellung des zeitlichen Verlaufs der Förderrate der Substituatpumpe, der Förderrate der Blutpumpe und des venösen Drucks in einer Dialysemaschine gemäß dem Stand der Technik; und
- Figur 3:: eine schematische Darstellung des zeitlichen Verlaufs der Förderrate der Substituatpumpe, der Förderrate der Blutpumpe und des venösen Drucks in einer erfindungsgemäßen Dialysemaschine.

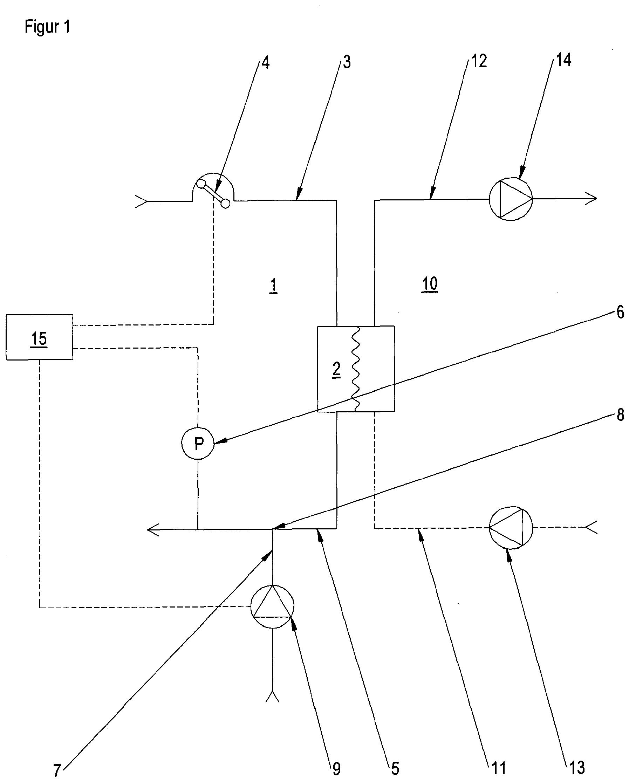

Figur 1 zeigt eine schematische Darstellung einer erfindungsgemäßen Dialysemaschine. Diese Dialysemaschine weist einen extrakorporalen Blutkreislauf 1 und einen Dialysator 2 auf. Bei dem Dialysator 2 kann es sich beispielsweise um einen Hohlfaserdialysator handeln.

In der arteriellen Leitung 3 des extrakorporalen Blutkreislaufs 1 befindet sich die Blutpumpe 4. Bei der Blutpumpe 4 kann es sich beispielsweise um eine peristaltische Pumpe handeln. In der venösen Leitung 5 des extrakorporalen Blutkreislaufs befindet sich ein venöser Drucksensor 6. Ferner ist eine Postdilutionsleitung 7 vorgesehen, die stromaufwärts des venösen Drucksensors 6 an der Mündungsstelle 8 in die venöse Leitung 5 des extrakorporalen Blutkreislaufs einmündet. In der Postdilutionsleitung 7 befindet sich die Substituatpumpe 9.

Der extrakorporale Blutkreislauf 1 steht über die im Dialysator 2 angeordnete Membran mit dem Dialysierflüssigkeitskreislauf (Dialysatkreislauf) 10 in Verbindung. Die gezeigte Ausführungsform der Dialysemaschine kann je nach Geräteeinstellung zur Durchführung einer Hämodialyse, einer Hämodiafiltration oder einer Hämofiltration ausgebildet sein. Im Fall der Hämodialyse und der Hämodiafiltration wird die Dialysatseite des Dialysators 2 über den stromaufwärts des Dialysators 2 befindlichen Teil 11 des Dialysatkreislaufs 10 mit frischer Dialysierflüssigkeit gespeist. Über den stromabwärts des Dialysators 2 befindlichen Teil 12 des Dialysatkreislaufs 10 fließt verbrauchtes Dialysat und ggf. Ultrafiltrat ab. Im Fall der Hämofiltration entfällt eine Speisung der Dialysatseite des Dialysators 2 mit Dialysierflüssigkeit und es wird lediglich Ultrafiltrat über den stromabwärts des Dialysators 2 befindlichen Teil 12 des Dialysatkreislaufs 10 abgeführt. Da der stromaufwärts des Dialysators 2 befindliche Teil 11 des Dialysatkreislaufs 10 nicht in jeder Betriebsform der Maschine in Verwendung steht, ist er in der Figur lediglich gepunktet dargestellt. Im gezeigten Ausführungsbeispiel sind im Dialysatkreislauf 10 sowohl stromaufwärts als auch stromabwärts des Dialysators 2 Dialysatpumpen 13 und 14 angeordnet. Selbstverständlich ist aber auch eine andere Anordnung dieser Pumpen oder ein Weglassen zumindest einer dieser Pumpen denkbar.

Die Postdilutionsleitung 7 zweigt entweder vom stromaufwärts des Dialysators 2 befindlichen Teil 11 des Dialysatkreislaufs 10 ab und bezieht somit das Substituat aus dieser Leitung, oder führt zu einem nicht näher dargestellten separaten Reservoir für das Substituat.

Die Steuereinheit 15 steht im gezeigten Ausführungsbeispiel mit der Blutpumpe 4 mit dem venösen Drucksensor 6 und mit der Substituatpumpe 9 in Verbindung. Sie regelt die Förderrate der Blutpumpe 4 und empfängt Signale des venösen Drucksensors 6. Im Normalbetrieb regelt die Steuereinheit 15 die Blutpumpe 4 so, dass eine bestimmte Förderrate für das Blut gewählt wird. Die Förderrate wird beispielsweise so gewählt, dass der Transmembrandruck am Dialysator 2 ein Erreichen der Behandlungsziele erlaubt. Details zur Wahl der Förderrate im Normalbetrieb sind im Stand der Technik bekannt und nicht Gegenstand der vorliegenden Patentanmeldung.

In der Steuereinheit 15 ist ein Algorithmus hinterlegt, der nach Erkennung eines Triggerereignisses temporär einen sogenannten speziellen Betriebsmodus der Blutpumpe 4 startet. In diesem speziellen Betriebsmodus wird die Förderrate der Blutpumpe in Abhängigkeit des am venösen Drucksensor 6 ermittelten venösen Drucks geregelt, und zwar derart, dass der gemessene venöse Druck mit einem Sollwert verglichen wird und die Pumpe in Abhängigkeit des Ergebnisses dieses Vergleichs geregelt wird. So wird die Förderrate der Blutpumpe im speziellen Betriebsmodus erniedrigt, wenn der gemessene venöse Druck den Sollwert übersteigt, und erhöht, wenn der gemessene venöse Druck unter dem Sollwert liegt.

Dieses Ausführungsbeispiel beschreibt also eine Regelung der Pumpenrate auf der Grundlage des venösen Drucks. Die Erfindung ist aber selbstverständlich nicht auf die Annahme des venösen Drucks als Sollwert beschränkt. Ferner ist die Erfindung selbstverständlich nicht auf die Regelung beschränkt, sondern kann an Stelle der Regelung auch eine Steuerung umfassen, die eine Vorgabe, beispielsweise einen venösen Druck oder eine Motorleistungsaufnahme der Blutpumpe unmittelbar vor dem speziellen Modus zur Bestimmung der Pumpenrate verwendet. Die im Zusammenhang mit dem Ausführungsbeispiel gemachten Angaben können selbstverständlich auch auf diese alternativen Ausführungsformen angewandt werden.

Im vorliegenden Ausführungsbeispiel wird der Sollwert aus einem vor dem Start des speziellen Modus am venösen Drucksensor 6 gemessenen Druck abgeleitet. Beispielsweise kann ein 1 Sekunde vor dem Start des speziellen Modus am venösen Drucksensor 6 gemessener Einzelwert als Sollwert herangezogen werden, wobei dies lediglich exemplarisch und diese Wahl des Sollwertes keinen notwendigen Bestandteil des vorliegenden Ausführungsbeispiels darstellt.

Als Triggerereignis wird im vorliegenden Ausführungsbeispiel eine Über- oder Unterschreitung eines Schwellenwertes für den am venösen Drucksensor 6 gemessenen Druck angenommen. Diese Wahl ist ebenfalls lediglich exemplarisch und stellt keinen notwendigen Bestandteil des vorliegenden Ausführungsbeispiels dar. Ebenso wäre es denkbar, dass die Steuereinheit 15 ferner mit der Substituatpumpe 9 in Verbindung steht und als Triggerereignis ein Stopp, eine Verringerung, ein Start oder eine Erhöhung der Substituatgabe gewählt wird. Diese Ereignisse korrespondieren beispielsweise zu einer Verlangsamung der Substituatpumpe 9, z.B. bei einem Zusetzen des Dialysators 2 (oft auch als Clotting bezeichnet), oder einem Stoppen der Substituatpumpe 9 ohne Auswirkungen auf die Blutpumpe 4, z.B. wenn eine Druckhaltetest der Hydraulik erfolgt, Alarme der Substituatversorgung ausgelöst werden (Leitfähigkeit oder Temperatur des Dialysats unphysiologisch) oder ein Wechsel der Behandlungsart stattfindet (Hämodiafiltration nach Hämodialyse, Hämodiafiltration nach Hämofiltration oder umgekehrt).

Die Dauer des speziellen Betriebsmodus entspricht im vorliegenden Ausführungsbeispiel der - unter Annahme der Förderrate vor Beginn des speziellen Betriebsmodus - prognostizierten Flusszeit des Blutes durch die venöse Leitung nebst Sicherheitszuschlag von 30%. Diese Wahl ist exemplarisch und stellt keinen notwendigen Bestandteil des vorliegenden Ausführungsbeispiels dar.

Erfindungsgemäß ist vorgesehen, dass die Steuereinheit anhand des hinterlegten Algorithmus nach einer Erkennung des Triggerereignisses noch eine weitere Abfrage durchführt, bevor der spezielle Betriebsmodus der Blutpumpe 4 gestartet wird. Diese Abfrage ist darauf gerichtet, ob ein Hinderungsgrund vorliegt, der gegen das Starten des speziellen Modus spricht oder zumindest eine Anpassung bei der Wahl des Sollwertes erfordert.

Ein derartiger Hinderungsgrund besteht darin, dass die Blutpumpe 4 aktuell bereits im speziellen Modus betrieben wird. In diesem Fall wird die Dauer des speziellen Betriebsmodus zwar verlängert und insoweit ein weiterer spezieller Betriebsmodus gestartet. Der Sollwert für den venösen Druck wird aber nicht anhand eines während des bestehenden speziellen Betriebsmodus - und somit 1 Sekunde vor Start des weiteren speziellen Betriebsmodus - ermittelten Messwertes für den venösen Druck festgelegt, sondern der Sollwert wird aus dem bestehenden speziellen Betriebsmodus übernommen.

Ein weiterer Hinderungsgrund besteht darin, dass die Blutpumpe 4 erst seit kurzer Zeit im Normalbetrieb läuft, beispielsweise deshalb, weil gerade erst eine Phase geendet hat, in der die Blutpumpe im speziellen Betriebsmodus betrieben wurde. Insoweit besteht dieser Hinderungsgrund beispielsweise in einer zu zeitnahen Abfolge zweier Phasen, in denen die Blutpumpe 4 im speziellen Betriebsmodus betrieben wird. In diesem Fall wird entweder die Auslösung des speziellen Betriebsmodus blockiert oder der Sollwert aus dem zeitlich vorausgehenden speziellen Betriebsmodus wird für den neuen speziellen Betriebsmodus angenommen. Die Dauer der Karenzzeit wird im vorliegenden Ausführungsbeispiel so gewählt, dass sie der prognostizierten Flusszeit des Blutes durch die venöse Leitung des extrakorporalen Blutkreislaufs entspricht. Die Flusszeit wird anhand der Förderrate der Blutpumpe vor Eintritt in den (vergangenen) speziellen Betriebsmodus und der bekannten Bauart der venösen Leitung prognostiziert. Diese Wahl ist exemplarisch und stellt keinen notwendigen Bestandteil des vorliegenden Ausführungsbeispiels dar. Beispielsweise kann auch einfach ein bestimmter Volumenstrom durch die Blutpumpe 4 oder einfach eine festgelegte Zeitdauer verwendet werden.

Ein weiterer Hinderungsgrund besteht darin, dass die erste zeitliche Ableitung des venösen Drucks bei Vorliegen des Triggerereignisses einen Schwellenwert überschreitet, d.h. wenn ein starker Drift vorliegt und das System nicht stabil ist. Sofern dies der Fall ist, ist fraglich, ob sich ein geeigneter Sollwert ermitteln lässt. Auch in diesem Fall wird entweder die Auslösung des speziellen Betriebsmodus blockiert oder der Sollwert wird aus dem zeitlich vorausgehenden speziellen Betriebsmodus für den neuen speziellen Betriebsmodus angenommen.

Die Blockierung der Auslösung des speziellen Betriebsmodus kann im Ergebnis auch zu einer Verschiebung der Auslösung des speziellen Betriebsmodus führen, nämlich dann, wenn das Triggerereignis (z.B. die Überschreitung des Grenzwertes für den venösen Druck) bestehen bleibt und der Hinderungsgrund wegfällt.

Im vorliegenden Ausführungsbeispiel werden diese Hinderungsgründe kumulativ abgefragt, es ist jedoch auch denkbar und von der Erfindung umfasst, wenn lediglich einer oder zwei dieser Hinderungsgründe abgefragt werden.

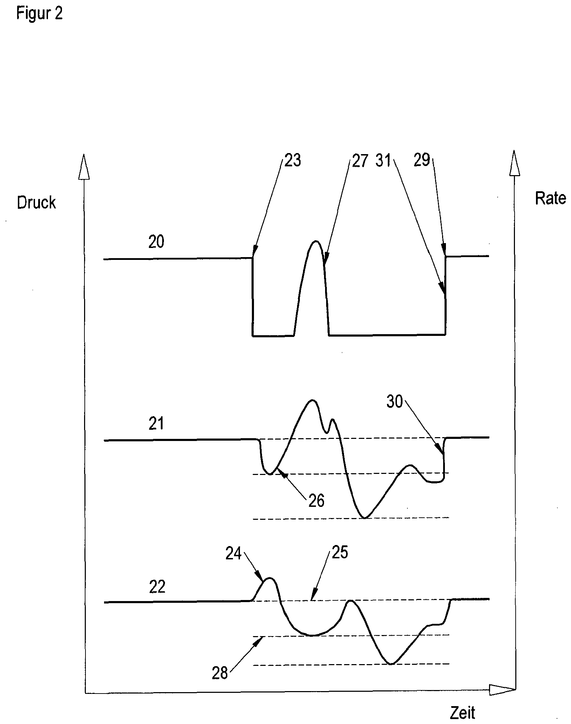

Figur 2 zeigt eine schematische Darstellung des zeitlichen Verlaufs der Förderrate der Substituatpumpe 20, der Förderrate der Blutpumpe 21 und des venösen Drucks 22 in einer Dialysemaschine gemäß dem Stand der Technik. Die Dialysemaschine kann baulich dieselben Elemente aufweisen, wie diese im Zusammenhang mit Figur 1 erklärt wurden. Gegenüber der Erfindung fehlt es lediglich an der Ausbildung der Steuereinheit dahingehend, dass sie nach einer Erkennung des Triggerereignisses noch eine weitere Abfrage durchführt, bevor der spezielle Betriebsmodus der Blutpumpe gestartet wird.

Wie dies der Figur zu entnehmen ist, wird im dargestellten Beispiel aus dem Stand der Technik eine Änderung der Substitutionsrate als Triggerereignis verwendet (Bezugszeichen 23). Beispielsweise führt vorliegend ein Druckhaltetest des Substituatsystems zum Triggern des speziellen Betriebsmodus. Der Ausfall der Substitution führt zum Vorliegen von dickerem Blut in der venösen Leitung und zum Ansteigen des venösen Drucks (Bezugszeichen 24). Da die Blutpumpe aber im speziellen Modus in Abhängigkeit des venösen Drucks derart kontrolliert wird, dass ein Verbleiben des venösen Drucks am Sollwert gewünscht wird (Bezugszeichen 25), erniedrigt sich mit steigendem venösen Druck die Förderrate der Blutpumpe (Bezugszeichen 26). Der Sollwert wird im Stand der Technik anhand des venösen Drucks vor Beginn des speziellen Modus festgelegt. Wie aus den Kurven 21 und 22 erkennbar ist, unterliegt der Druckverlauf während des speziellen Betriebsmodus einer gewissen Trägheit. Insoweit fluktuiert der tatsächliche venöse Druck um den Sollwert.

Kommt es nun zu einem weiteren Triggerereignis noch während der spezielle Betriebsmodus besteht (beispielsweise in Form einer Bolusgabe, Bezugszeichen 27), wird ein neuer spezieller Modus gestartet. Dieser spezielle Modus unterscheidet sich vom bestehenden speziellen Modus im Betrag des Sollwertes, der anhand des venösen Drucks vor Beginn des nunmehrigen weiteren speziellen Modus festgelegt wird (Bezugszeichen 28). Durch die oben beschriebene Fluktuation weicht dieser Sollwert aber in signifikantem Ausmaß von dem venösen Druck ab, der im Normalbetrieb der Blutpumpe zu erwarten wäre. Insoweit erfolgt mit Blick auf die Figuren ein Sinken der Blutpumpenrate auf die unterste gestrichelte Linie, was auch ein Sinken des venösen Drucks auf die gestrichelte Linie zur Folge hat. Denn als Sollwert wird nicht, was korrekt wäre, der Anfangswert vor dem ersten Ereignis angenommen, sondern derjenige, der fälschlicherweise vom System als Ausgangswert angenommen wird, also gemäß Figur 2 derjenige Wert auf der mittleren Linie der Kurve 21.

Dies führt dazu, dass zum Ende des speziellen Modus (Bezugszeichen 29) die Blutpumpenrate stark ansteigt (Bezugszeichen 30). Da die Rate der Substituatpumpe im System gemäß Figur 2 an die Rate der Blutpumpe gekoppelt ist, steigt zu Ende des speziellen Betriebsmodus auch die Rate der Substitutionspumpe schlagartig (Bezugszeichen 31). Dies kann zu einer unerwünschten Selbsttriggerung des speziellen Modus führen.

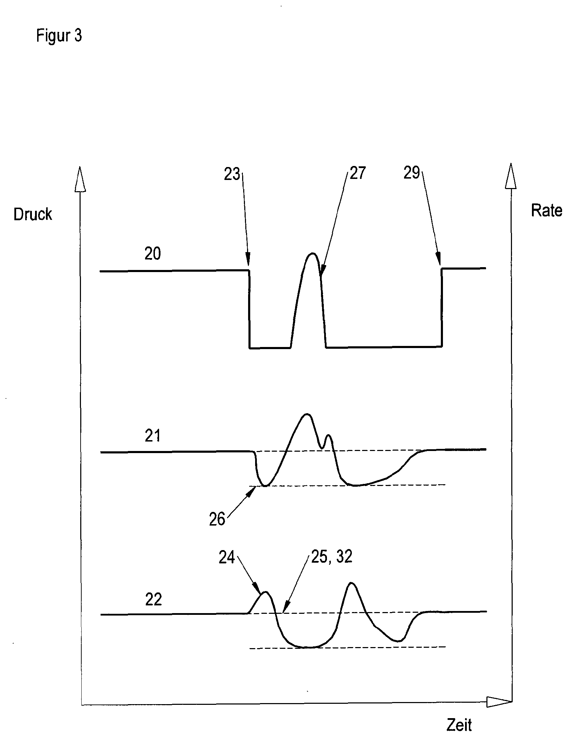

Figur 3 zeigt eine schematische Darstellung des zeitlichen Verlaufs der Förderrate der Substituatpumpe 20, der Förderrate der Blutpumpe 21 und des venösen Drucks 22 in einer erfindungsgemäßen Dialysemaschine. Die Dialysemaschine ist identisch zur Dialysemaschine aufgebaut, die der Darstellung gemäß Figur 2 zu Grunde liegt. Lediglich ist hier vorgesehen, dass die Steuereinheit nach einer Erkennung des Triggerereignisses noch eine weitere Abfrage durchführt, bevor der spezielle Betriebsmodus der Blutpumpe gestartet wird.

In der Figur 3 werden gegenüber der Figur 2 für entsprechende Ereignisse bzw. Merkmale entsprechende Bezugszeichen verwendet. Auch die dazugehörige Erklärung kann entsprechend übernommen werden.

Wie dies aus der Figur zu erkennen ist, wird nach Erkennung des zweiten Triggerereignisses (Bezugszeichen 27) von der Steuereinheit vor dem Start eines weiteren speziellen Modus eine Abfrage nach dem Vorliegen eines Hinderungsgrundes durchgeführt. Ein solcher Hinderungsgrund wird dahingehend erkannt, dass die Blutpumpe aktuell bereits im speziellen Modus betrieben wird. Infolgedessen wird für den zweiten speziellen Modus nicht ein neuer Sollwert für den venösen Druck gewählt, wie dies in Figur 2 der Fall war (dort: Bezugszeichen 28), sondern der Sollwert wird aus dem bestehenden speziellen Betriebsmodus übernommen (Bezugszeichen 32). Dieser Sollwert entspricht ungefähr dem venösen Druck, der im Normalbetrieb der Blutpumpe zu erwarten wäre. Dies führt dazu, dass zum Ende des speziellen Modus keine signifikante Änderung der Förderrate der Blutpumpe erforderlich ist und eine unerwünschte Selbsttriggerung des Systems vermieden wird.

## Patentansprüche

1. Dialysemaschine mit einem extrakorporalen Blutkreislauf (1), einer Blutpumpe (4), einem Dialysator (2), einem venösen Drucksensor (6), einer Substituatleitung (7) und einer Steuereinheit (15), wobei die Steuereinheit (15) ausgebildet ist, die Blutpumpe (4) in einem ersten Betriebsmodus und einem speziellen Betriebsmodus zu betreiben und nach Erkennung eines Triggerereignisses (23) den speziellen Betriebsmodus zu starten, in dem eine Förderrate (21) der Blutpumpe (4) anhand einer Vorgabe gesteuert oder auf einen Sollwert (25) geregelt wird, die bzw. der aus einem vor Beginn des aktuell gestarteten speziellen Betriebsmodus ermittelten Wert abgeleitet wird oder diesem entspricht,
**dadurch gekennzeichnet,**
**dass** die Steuereinheit (15) ferner so ausgebildet ist, dass vor Start des speziellen Betriebsmodus das Vorliegen wenigstens eines Hinderungsgrundes abgefragt wird, und dass bei Vorliegen des Hinderungsgrundes der Start des speziellen Betriebsmodus blockiert oder verschoben wird und/oder die Wahl der Vorgabe bzw. des Sollwertes (25) bei Vorliegen des Hinderungsgrundes von der Wahl ohne Vorliegen des Hinderungsgrundes abweicht.

2. Dialysemaschine nach Anspruch 1, **dadurch gekennzeichnet, dass** der vor Beginn des aktuell gestarteten speziellen Betriebsmodus ermittelte Wert, der Sollwert (25) oder die Vorgabe eine Bluteigenschaft, wie etwa eine Blutviskosität oder ein Hämatokritanteil, oder ein Gerätewert, wie etwa eine Motorstromaufnahme oder Motorleistungsaufnahme der Blutpumpe (4) oder ein venöser Druck ist.

3. Dialysemaschine nach einem der vorhergehenden Ansprüche, **dadurch gekennzeichnet, dass** ein Hinderungsgrund darin besteht, dass die Blutpumpe (4) aktuell bereits im speziellen Modus betrieben wird.

4. Dialysemaschine nach Anspruch 3, **dadurch gekennzeichnet, dass** die Vorgabe bzw. der Sollwert (25) aus dem bestehenden speziellen Betriebsmodus übernommen wird, wenn die Blutpumpe (4) aktuell bereits im speziellen Modus betrieben wird.

5. Dialysemaschine nach einem der vorhergehenden Ansprüche, **dadurch gekennzeichnet, dass** wenigstens einer der Hinderungsgründe darin besteht, dass eine bestimmte Karenzzeit nach Start oder Ablauf einer zeitlich vorgelagerten Phase, in der die Blutpumpe (4) im speziellen Betriebsmodus betrieben wurde, noch nicht erreicht wurde.

6. Dialysemaschine nach Anspruch 5, **dadurch gekennzeichnet, dass** die Vorgabe bzw. der Sollwert (25) aus dem speziellen Betriebsmodus während der zeitlich vorgelagerten Phase übernommen wird.

7. Dialysemaschine nach einem der vorhergehenden Ansprüche, **dadurch gekennzeichnet, dass** ein Hinderungsgrund darin besteht, dass die erste zeitliche Ableitung eines Messwertes, beispielsweise des venösen Drucks bei Vorliegen des Triggerereignisses (23) einen Schwellenwert überschreitet.

8. Dialysemaschine nach einem der vorhergehenden Ansprüche, **dadurch gekennzeichnet, dass** die Vorgabe bzw. der Sollwert (25) aus dem Messwert, beispielsweise dem venösen Druck vor Starten des speziellen Modus abgeleitet wird.

9. Dialysemaschine nach einem der vorhergehenden Ansprüche, **dadurch gekennzeichnet, dass** die Steuereinheit (15) so ausgebildet ist, dass die Blutpumpe (4) vor und/oder nach dem speziellen Betriebsmodus mit einem vorgegebenen Flussratenverlauf, insbesondere bei konstanter Flussrate oder konstanter Drehzahl betrieben wird.

10. Dialysemaschine nach einem der vorhergehenden Ansprüche, **dadurch gekennzeichnet, dass** das Triggerereignis (23) in der Über- oder Unterschreitung eines Schwellenwertes oder Gradienten für einen Messwert, beispielsweise den venösen Drucks und/oder in einem Stopp, einer Verringerung, einem Start oder einer Erhöhung der Substituatgabe liegt.

11. Dialysemaschine nach einem der vorhergehenden Ansprüche, **dadurch gekennzeichnet, dass** die Steuereinheit (15) so ausgebildet ist, dass die Dauer des speziellen Betriebsmodus an die prognostizierte Flusszeit des Blutes durch die venöse Leitung des extrakorporalen Blutkreislaufs (1) unter Normalbetrieb der Blutpumpe (4) angenähert wird oder sich daran orientiert.

12. Dialysemaschine nach einem der vorhergehenden Ansprüche, **dadurch gekennzeichnet, dass** die Steuereinheit (15) so ausgebildet ist, dass die Karenzzeit mit der Dauer des speziellen Betriebsmodus korreliert.

13. Dialysemaschine nach einem der vorhergehenden Ansprüche, **dadurch gekennzeichnet, dass** die Leitung für die Zufuhr von Substituat in der venösen Leitung des extrakorporalen Blutkreislaufs (1) mündet.

14. Dialysemaschine nach einem der vorhergehenden Ansprüche, **dadurch gekennzeichnet, dass** die Steuereinheit (15) ferner so ausgebildet ist, dass während des Verlaufs des speziellen Betriebsmodus eine Veränderung in der Zufuhrrate des Substituats blockiert oder nur innerhalb eines vorgegebenen Bereichs zugelassen wird.

15. Dialysemaschine nach einem der vorhergehenden Ansprüche, **dadurch gekennzeichnet, dass** die Steuereinheit (15) ferner so ausgebildet ist, dass innerhalb einer gewissen Wartezeit nach Ende des speziellen Betriebsmodus eine Veränderung der Zufuhr des Substituats blockiert oder nur innerhalb eines vorgegebenen Bereichs zugelassen wird.

## Claims

1. A dialysis machine having an extracorporeal blood circuit (1), a blood pump (4), a dialyzer (2), a venous pressure sensor (6), a substituate line (7) and a control unit (15), wherein the control unit (15) is configured to operate the blood pump (4) in a first operating mode and in a special operating mode and to start the special operating mode after recognition of a trigger event (23), in which special operating mode a conveying rate (21) of the blood pump (4) is controlled by means of a default value or is regulated to a desired value (25), which default or desired value is derived from a value determined before the start of the currently started special operating mode or corresponds to said value,
**characterized in that**
the control unit (15) is furthermore configured such that the presence of at least one obstacle is polled before the start of the special operating mode, and **in that**, on the presence of the obstacle, the start of the special operating mode is blocked or delayed and/or the selection of the default value or of the desired value (25) on the presence of the obstacle differs from the selection without the presence of the obstacle.

2. A dialysis machine in accordance with claim 1, **characterized in that** the value determined before the start of the currently started special operating mode, the desired value (25) or the default value, is a blood property such as a blood viscosity or a hematocrit portion, or an instrument value such as a motor current draw or a motor power draw of the blood pump (4), or a venous pressure.

3. A dialysis machine in accordance with one of the preceding claims, **characterized in that** an obstacle consists **in that** the blood pump (4) is currently already being operated in the special mode.

4. A dialysis machine in accordance with claim 3, **characterized in that** the default value or the desired value (25) is taken over from the existing special operating mode when the blood pump (4) is currently already being operated in the special mode.

5. A dialysis machine in accordance with one of the preceding claims, **characterized in that** at least one of the obstacles consists **in that** a specific waiting period after the start or elapse of a phase which is deployed beforehand in time and in which the blood pump (4) is operated in the special operating mode has not yet been reached.

6. A dialysis machine in accordance with claim 5, **characterized in that** the default value or the desired value (25) is taken over from the special operating mode during the phase deployed beforehand in time.

7. A dialysis machine in accordance with one of the preceding claims, **characterized in that** an obstacle consists **in that** the first time derivative of a measured value, for example the venous pressure on the presence of a trigger event (23), exceeds a threshold value.

8. A dialysis machine in accordance with one of the preceding claims, **characterized in that** the default value or the desired value (25) is derived from the measured value, for example from the venous pressure before starting the special mode.

9. A dialysis machine in accordance with one of the preceding claims, **characterized in that** the control unit (15) is configured such that the blood pump (4) is operated before and/or after the special operating mode at a predefined flow rate development, in particular at a constant flow rate or at a constant speed.

10. A dialysis machine in accordance with one of the preceding claims, **characterized in that** the trigger event (23) is the exceeding or falling below of a threshold value or gradient for a measured value, for example the venous pressure and/or a stop, a reduction, a start or an increase in the substituate administration.

11. A dialysis machine in accordance with one of the preceding claims, **characterized in that** the control unit (15) is configured such that the duration of the special operating mode is approximated to or orientated on the forecast flow time of the blood through the venous line of the extracorporeal blood circuit (1) under normal operation of the blood pump (4).

12. A dialysis machine in accordance with one of the preceding claims, **characterized in that** the control unit (15) is configured such that the waiting period correlates with the duration of the special operating mode.

13. A dialysis machine in accordance with one of the preceding claims, **characterized in that** the line for the supply of substituate opens in the venous line of the extracorporeal blood circuit (1).

14. A dialysis machine in accordance with one of the preceding claims, **characterized in that** the control unit (15) is furthermore configured such that, during the course of the special operating mode, a change in the supply rate of the substituate is blocked or is only permitted within a predefined range.

15. A dialysis machine in accordance with one of the preceding claims, **characterized in that** the control unit (15) is furthermore configured such that, within a certain waiting period after the end of the special operating mode, a change in the supply of the substituate is blocked or is only permitted within a predefined range.

## Revendications

1. Machine de dialyse avec un circuit de sang extracorporel (1), une pompe à sang (4), un dialyseur (2), un capteur de pression veineuse (6), une conduite de solution de substitution (7) et une unité de commande (15), ladite unité de commande (15) étant conçue de manière à faire fonctionner la pompe à sang (4) dans un premier mode de fonctionnement et dans un mode de fonctionnement spécial et, après reconnaissance d'un événement déclencheur (23), à démarrer le mode de fonctionnement spécial dans lequel un débit (21) de la pompe à sang (4) est contrôlé au moyen d'une valeur prédéfinie ou réglé sur une valeur de consigne (25), laquelle est dérivée à partir d'une valeur déterminée avant le début du mode de fonctionnement spécial présentement en cours, ou qui lui correspond,
**caractérisée en ce que**
l'unité de commande (15) est par ailleurs conçue de sorte, qu'avant le démarrage du mode de fonctionnement spécial, l'existence d'au moins un empêchement est vérifiée et **en ce que**, si cet empêchement existe, le démarrage du mode de fonctionnement spécial est bloqué ou reporté et/ou le choix de la valeur prédéfinie voire de la valeur de consigne (25) diffère, en cas d'empêchement, du choix lorsqu'il n'existe aucun empêchement.

2. Machine de dialyse selon la revendication 1, **caractérisée en ce que** la valeur déterminée avant le démarrage du mode de fonctionnement spécial en cours, la valeur de consigne (25) ou la valeur prédéfinie est une caractéristique du sang, comme une viscosité du sang ou un taux d'hématocrite, ou une valeur de l'appareil, comme la consommation de courant du moteur ou la puissance moteur de la pompe à sang (4) ou encore une pression veineuse.

3. Machine de dialyse selon l'une quelconque des revendications précédentes, **caractérisée en ce qu'**il existe un empêchement qui réside dans le fait que la pompe à sang (4) fonctionne déjà actuellement dans un mode spécial.

4. Machine de dialyse selon la revendication 3, **caractérisée en ce que** la valeur prédéfinie voire la valeur de consigne (25) est reprise depuis le mode de fonctionnement spécial lorsque la pompe à sang (4) fonctionne déjà actuellement dans un mode spécial.

5. Machine de dialyse selon l'une quelconque des revendications précédentes, **caractérisée en ce qu'**au moins l'un des empêchements réside dans le fait qu'un délai de carence spécifique n'a pas encore été atteint après le démarrage ou l'expiration d'une phase en amont, durant laquelle la pompe à sang (4) a été utilisée dans le mode de fonctionnement spécial.

6. Machine de dialyse selon la revendication 5, **caractérisée en ce que** la valeur prédéfinie voire la valeur de consigne (25) dérivée à partir du mode de fonctionnement spécial est reprise pendant la phase en amont.

7. Machine de dialyse selon l'une quelconque des revendications précédentes, **caractérisée en ce qu'**un empêchement réside dans le fait que la première dérivée de temps d'une valeur de mesure, par exemple de la pression veineuse, dépasse une valeur seuil en présence d'un événement déclencheur (23).

8. Machine de dialyse selon l'une quelconque des revendications précédentes, **caractérisée en ce que** la valeur prédéfinie ou la valeur de consigne (25) est dérivée à partir de la valeur de mesure, par exemple de la pression veineuse avant le démarrage du mode spécial.

9. Machine de dialyse selon l'une quelconque des revendications précédentes, **caractérisée en ce que** l'unité de commande (15) est conçue de sorte que la pompe à sang (4) est actionnée avant et/ou après le mode de fonctionnement spécial avec une allure de débit prescrite, en particulier en cas de débit constant ou de vitesse constante.

10. Machine de dialyse selon l'une quelconque des revendications précédentes, **caractérisée en ce que** l'événement déclencheur (23) consiste en un dépassement par le haut ou par le bas d'une valeur seuil ou d'un gradient pour une valeur de mesure, par exemple la pression veineuse et/ou en un arrêt, une diminution, un démarrage ou une augmentation de l'administration de la solution de substitution.

11. Machine de dialyse selon l'une quelconque des revendications précédentes, **caractérisée en ce que** l'unité de commande (15) est conçue de sorte que la durée du mode de fonctionnement spécial se rapproche ou s'oriente sur le temps d'écoulement du sang pronostiqué à travers la conduite veineuse du circuit de sang extracorporel (1) dans des conditions de fonctionnement normal de la pompe à sang (4).

12. Machine de dialyse selon l'une quelconque des revendications précédentes, **caractérisée en ce que** l'unité de commande (15) est conçue de sorte que le délai de carence est en corrélation avec la durée du mode de fonctionnement spécial.

13. Machine de dialyse selon l'une quelconque des revendications précédentes, **caractérisée en ce que** la conduite pour l'apport de la solution de substitution débouche dans la conduite veineuse du circuit de sang extracorporel (1).

14. Machine de dialyse selon l'une quelconque des revendications précédentes, **caractérisée en ce que** l'unité de commande (15) est par ailleurs conçue de sorte que, pendant le déroulement du mode de fonctionnement spécial, une modification du débit de la solution de substitution est bloquée ou n'est autorisée que dans les limites d'une plage prédéterminée.

15. Machine de dialyse selon l'une quelconque des revendications précédentes, **caractérisée en ce que** l'unité de commande (15) est par ailleurs conçue de sorte que, dans un temps d'attente spécifique après la fin du mode de fonctionnement spécial, une modification de l'apport de solution de substitution est bloquée ou uniquement autorisée dans les limites d'une plage prédéterminée.
